## Europäisches Patentamt

## European Patent Office

## Office européen des brevets

(19)

(11) Veröffentlichungsnummer: **0 078 971**
**B1**

(12) **EUROPÄISCHE PATENTSCHRIFT**

(45) Veröffentlichungstag der Patentschrift:
27.03.85

(51) Int. Cl.⁴: **C 12 Q 1/54**

(21) Anmeldenummer: 82109853.0

(22) Anmeldetag: 26.10.82

(54) Mittel zum Nachweis von Glucose in biologischen Flüssigkeiten.

(30) Priorität: 29.10.81 DE 3142862

(43) Veröffentlichungstag der Anmeldung:
18.05.83 Patentblatt 83/20

(45) Bekanntmachung des Hinweises auf die Patenterteilung:
27.03.85 Patentblatt 85/13

(84) Benannte Vertragsstaaten:
AT CH DE FR GB IT LI

(56) Entgegenhaltungen:
DE - A - 2 941 299
US - A - 3 964 871

CLINICAL CHEMISTRY, Band 27, Nr. 7, Juli 1981 Easton, Pennsylvania, USA
BIOCHEMICAL JOURNAL, Band 42, 1948, Cambridge D. KEILIN et al. "Properties of glucose oxidase (notatin)"

(73) Patentinhaber: BEHRINGWERKE AKTIENGESELLSCHAFT, Postfach 1140, D-3550 Marburg/Lahn (DE)

(72) Erfinder: Kohl, Helmut, Dr., Goethestrasse 32, D-3552 Wetter (DE)

(74) Vertreter: Meyer-Dulheuer, Karl-Hermann, Dr. et al, HOECHST Aktiengesellschaft Zentrale Patentabteilung Postfach 80 03 20, D-6230 Frankfurt/Main 80 (DE)

## Beschreibung

Die Erfindung betrifft ein Mittel zum Nachweis von Glucose in biologischen Flüssigkeiten, das im wesentlichen aus einer adsorbierenden Matrix, einer Glucoseoxidase, einer Peroxidase und einem Chromogen als Reagenz besteht und ein Nitrat und gegebenenfalls einen UV-Absorber enthält.

Der Diabetis mellitus ist eine chronische Krankheit, die fortwährend überwacht werden muß. Es hat sich als sinnvoll erwiesen, daß der Diabetiker nach Einweisung durch den Arzt seine Stoffwechsellage selber kontrolliert, um so gegebenenfalls sofort die notwendige Insulin-Dosis dem wechselnden Bedarf anpassen zu können. Die Stoffwechselkontrolle wird so durchgeführt, daß der Glucose-Gehalt routinemäßig im Harn oder im Blut bestimmt wird. Im allgemeinen wird dabei vom Diabetiker der Harn-Glucose-Wert selber halbquantitativ bestimmt, während die quantitative Blutzucker-Messung dagegen vom Fachmann durchgeführt wird.

Bei der Harn-Glucose-Bestimmung sollte ein Konzentrationsbereich bis etwa 5 g Glucose/100 ml Harn erfaßt werden.

Zur Zeit gibt es zwei Methoden für die schnelle halbquantitative Glucose-Bestimmung:

1.  Test-Tabletten entsprechend der unspezifischen Reduktionsmethode nach BENEDIKT und

2.  Teststreifen, die mit der spezifisch reagierenden Glucose-Oxidase, einem Chromogen und einer Peroxidase imprägniert sind.

In den Teststreifen katalysiert die Glucoseoxidase die Oxidation von Glucose mit Sauerstoff zu Gluconolacton und Wasserstoffperoxid. In einer unspezifischen nachgeschalteten Reaktion oxydiert dieses unter Peroxidase-Katalyse ein Chromogen zu einem Farbstoff.

Die beiden erwähnten Methoden sind mit Nachteilen behaftet:

Die Reduktionsmethode ist unspezifisch, da auch andere reduzierende Verbindungen wie beispielsweise Galactose erfaßt werden. Weiterhin muß bei ihr mit ätzenden Reagenzien gearbeitet werden (G. Friedrich, »Der informierte Arzt« 8 (9), Separatum, 1980). Die Reaktion ist außerdem bei einer nicht unerheblichen Wärmetönung mit einer störenden Schaumbildung verbunden. Die Handhabung der Teste mit Tabletten und Reagenzgläsern ist aufwendig, umständlich und teuer.

Ähnliches gilt auch für die zweite Bestimmungsmethode. Da die semiquantitativen Urinteststreifen bisher nur einen Anzeigenbereich bis maximal 2 g Glucose/100 ml Urin umfassen, sind diese für die tagtägliche Routineuntersuchung nicht geeignet, weil höhere Glucose-Konzentrationen ebenfalls erfaßt werden sollen.

Zur Umgehung dieses Nachteils wird die Verwendung eines Verdünnungsbestecks vorgeschlagen (Sorge, Schlebusch, »Medizinische Klinik« 75, 587—591, 1980). Solche Verdünnungsbestecke sind für die einfache Routinebestimmung von Nachteil, da vom ungeübten Diabetiker Manipulationen erwartet werden, die er nicht beherrscht (Pipettieren, Verdünnen, Auswerten). Die Anforderungen an die Hygiene sind sehr hoch und nicht immer erfüllbar.

Dem Diabetiker sollte demgegenüber ein einfacher, leicht zu beherrschender und hygienisch einwandfreier Test zur Verfügung gestellt werden, der einen möglichst großen Konzentrationsbereich umfaßt.

Das Ziel der vorliegenden Erfindung war somit ein verbessertes Mittel zur Bestimmung von Glucose in biologischen Flüssigkeiten.

Aus Biochem. Journal 42, 221 (1948) ist bekannt, daß die Glucoseoxidase zu 13% von Natriumnitrat inhibiert wird. Eine Inhibierung der Glucoseoxidase wird ebenfalls in DE-A 2 941 299 beschrieben. Diese Hemmwirkung ist für Glucose-Teststreifen, bei denen mit einem großen Überschuß an Enzymen gearbeitet wird, so gering, daß von ihr bisher noch kein Gebrauch gemacht wurde. Eigene Versuche ergaben auch, daß ein entsprechender Schnelltest zur Glucose-Bestimmung von Nitraten nicht inhibiert wird.

Dagegen wurde nun überraschend gefunden, daß durch die Imprägnierung von Glucose-Teststreifen mit Nitraten deren Anzeigebereich wesentlich erweitert wird, besonders wenn hydrophierende Filme (H. Wishinsky in Quality Control in Clinical Chemistry; Amido, Rosalki, v. Kampen, S. 425, de Gruyter, Berlin 1975) verwendet werden. Es gelingt so, den Anzeigebereich eines üblichen semiquantitativen Testbezirks, der bis maximal etwa 2 g Glucose/100 ml Harn reicht, bis auf über 5 g Glucose/100 ml Harn zu erweitern. Diese modifizierten Teststreifen entsprechen somit bezüglich ihres Anwendungsbereiches den Anforderungen, die ein Diabetiker an ein einfaches Diagnostikum stellt. Weiterhin erfüllt ein solcher Teststreifen die hygienischen Ansprüche.

Es wurde weiterhin gefunden, daß durch den Zusatz von UV-Absorbern die Lichtbeständigkeit dieser Teststreifen verbessert werden kann.

Die oben geschilderte Aufgabe wird nun dadurch gelöst, daß das Mittel neben den üblichen Bestandteilen ein Nitrat und gegebenenfalls einen UV-Absorber enthält.

Gegenstand der Erfindung ist demnach ein Mittel zum Nachweis von Glucose in biologischen Flüssigkeiten, bestehend im wesentlichen aus einer adsorbierenden Matrix, enthaltend ein Chromogen, eine Glucoseoxidase, eine Peroxidase und gegebenenfalls einen Stabilisator, dadurch gekennzeichnet, daß es ein Nitrat und gegebenenfalls einen UV Absorber enthält.

Im weiteren Sinne ist Gegenstand der Erfindung die Verwendung von Ammoniumnitrat oder eines Nitrats eines Elements aus der ersten Gruppe des Periodensystems (erste Hauptgrup pe 1a CRC HANDBOOK of CHEMISTRY and PHYSICS 59th edition CRC Press 1978—1979), wobei Ammonium- und Natriumnitrat bevorzugt werden. Als UV-Absorber können verwendet werden beispielsweise die Derivate des Benzophenons, der Benzoesäure, Oxalsäure oder des Benzotriazins.

Verbindungen dieser Art werden beschrieben in »Ullmanns Enzyklopädie der technischen Chemie, 4. Auflage, Band 15, S. 259, Verlag Chemie, Weinheim, Bundesrepublik Deutschland. Bevorzugt werden 2,2'-Dihydroxy-4-methoxybenzophenon (Cyasorb® UV 24, American Cyanamid), 2-Hydroxy-4-methoxybenzophenonsulfonsäure-(5) (UV-Absorber HMBS »Riedel«, Riedel-de Haen AG, Seelze, Bundesrepublik Deutschland) oder Dihydroxybenzophenone.

Das erfindungsgemäße Mittel besteht somit aus einer adsorbierenden Trägermatrix, in welche eine Glucoseoxidase, eine Peroxidase, ein Chromogen, ein wasserlösliches Nitrat und gegebenenfalls ein UV-Absorber eingelagert sind. Als Trägermatrix können alle saugfähigen, flächenförmigen Gebilde natürlichen oder synthetischen Ursprungs verwendet werden wie beispielsweise Vliese, Papiere, Asbest oder Folien aus Polymeren.

Das Mittel wird zweckmäßigerweise so zubereitet, daß das Chromogen, der Puffer und die Enzyme in wäßriger Lösung gemeinsam mit dem Nitrat auf das Testpapier aufgetragen werden. In dieser Tränklösung kann der UV-Absorber ebenfalls gelöst werden. Es ist auch möglich, den UV-Absorber mit einem Hydrophobierungsmittel in einem weiteren Schritt auf die Trägermatrix aufzubringen. Die Reihenfolge der Tränkschritte ist beliebig; es kann auch mit der Hydrophobierung begonnen werden. Bewährt hat sich ein Verfahren, bei dem zunächst ein Indikatorrohpapier mit Chromogen, Puffer, Enzymen, Netzmittel, Stabilisator, einem wasserlöslichen Nitrat und einem UV-Absorber imprägniert wird. Dann werden ein weiterer UV-Absorber und ein Hydrophobierungsmittel, beispielsweise Ethylcellulose, in einem organischen mit Wasser nicht mischbaren Lösungsmittel, wie Toluol, gelöst aufgetragen.

Das bevorzugte Verfahren zur Herstellung eines erfindungsgemäßen Mittels umfaßt somit zwei Stufen. Nach dem Trocknen wird der Reaktionsträger zwecks leichterer Handhabung auf einen Kunststoff-Film laminiert.

Die Menge des jeweils verwendeten Nitrats ist nicht kritisch; sie kann dem jeweiligen Verwendungszweck, das heißt dem zu umfassenden Konzentrationsbereich angepaßt werden. Bezogen auf Ammoniumnitrat kann sie zwischen 0,1 und 7% (v : v) liegen. Bevorzugt wird eine Konzentration zwischen 0,3 und 4,0% (v : v).

Auch die Menge der verwendeten UV-Absorber ist nicht kritisch. Im allgemeinen wird für eine größere Nitratmenge auch eine größere Menge UV-Absorber eingesetzt. Die maximale Konzentration des UV-Absorbers ist durch dessen Löslichkeit in dem ausgewählten Lösungsmittel begrenzt. Die Konzentrationen können schwanken zwischen 0,1 und 5,0% (w : v), bevorzugt wird aber ein Konzentrationsbereich zwischen 0,4 und 3,0% (w : v).

Zur Verwendung des beschriebenen Mittels wird dieses kurz in die zu untersuchende Flüssigkeit eingetaucht. Abhängig von dem Gehalt der Flüssigkeit an Glucose entwickelt sich eine abgestufte Farbreaktion, die bis 5 g Glucose/100 ml Urin semiquantitativ abstufbar ist.

Bei vergleichenden Untersuchungen mit Schnelldiagnostika gemäß dem Stand der Technik zeigt sich der Vorteil der Erfindung.

Gemäß den Beispielen 1 bis 3 wurden drei Teststreifen-Papiere hergestellt, von denen 1a den Stand der Technik wiedergibt. 1b wurde unter Verwendung eines wasserlöslichen Nitrats hergestellt. 1c entspricht 1b, jedoch wurde das Papier zusätzlich mit einem UV-Absorber imprägniert.

Mit den konfektionierten Papieren wurden folgende Versuche durchgeführt:

Die Papiere 1a, 1b und 1c wurden dem Sonnenlicht ausgesetzt. 1b verfärbt sich hierbei in etwa 20 min. so stark grün, daß es nach dem anschließenden Benetzen mit einer Glucosefreien Lösung falsch positiv interpretiert wird. Die Papiere 1a und 1c verändern dagegen auch nach 60 min. ihre Grundfarbe nicht.

In einer weiteren Versuchsreihe wurden die Papiere 1a und 1c in Urine eingetaucht, die 50, 150, 500, 1500, 3000 und 5000 mg Glucose/100 ml Urin enthalten. Mit dem Papier 1a können die Konzentrationsstufen bis 1500 mg/dl gut unterschieden werden, die beiden höheren Glucose-Konzentrationen werden nicht mehr erkannt. Dagegen kann mit Papier 1c das gesamte Spektrum von 50 mg Glucose/100 ml Urin bis 5000 mg Glucose/100 ml Urin unterschieden werden.

Mit den folgenden Beispielen wird die Erfindung näher erläutert.

### Beispiel 1 / Papier 1a

Lösung 1    In 100 ml 0,1 M Citratpuffer pH 5,5 werden gelöst

        0,5 g    Gelatine
        7,0 mg Tatrazin
        0,6 g    o-Tolidin-di-hydrochlorid
        0,2 g    Peroxidase
        0,3 g    Glucoseoxidase
        1,0 g    Polyethylenglycol 1500.

        Papier 2316 von Schleicher und Schüll, Dassel, Bundesrepublik Deutschland, wird mit dieser Tränklösung imprägniert. Nach dem Trocknen bei 80° C im Trockenschrank wird das Papier nachimprägniert mit

Lösung 2    die 0,6 g Ethylcellulose in 100 ml Toluol enthält.

### Beispiel 2 / Papier 1b

Entsprechend Beispiel 1 wird ein Papier 1b so hergestellt, daß in der Lösung 1 noch 2 g Ammoniumnitrat gelöst werden. Die Nachimprägnierung erfolgt mit Lösung 2.

### Beispiel 3 / Papier 1c

In 100 ml der Tränklösung 1 gemäß Beispiel 1 werden noch

2,0 g Ammoniumnitrat und
1,0 g UV-Absorber HMBS »Riedel«

gelöst.
In 100 ml Toluol werden für die Nachimprägnierung

0,5 g Ethylcellulose und
2,0 g Cyasorb, Cyanamid,

gelöst.
Die Papiere 1a, 1b und 1c zeigen die früher beschriebenen Eigenschaften.

### Patentansprüche

1. Mittel zum Nachweis von Glucose in biologischen Flüssigkeiten, bestehend im wesentlichen aus einer adsorbierenden Matrix enthaltend ein Chromogen, eine Glucoseoxidase, eine Peroxidase und gegebenenfalls einen Stabilisator, dadurch gekennzeichnet, daß es ein Nitrat und gegebenenfalls einen UV-Absorber enthält.

2. Mittel nach Anspruch 1, dadurch gekennzeichnet, daß das Nitrat ein Nitrat eines Elements aus der ersten Gruppe des Periodensystems (erste Hauptgruppe 1a CRC HANDBOOK of CHEMISTRY and PHYSICS 59th edition CRC Press 1978—1979) oder Ammoniumnitrat ist.

3. Mittel nach Anspruch 1, dadurch gekennzeichnet, daß der UV-Absorber ein Derivat der Benzoesäure, des Benzophenons, der Oxalsäure oder ein Benzotriazin ist.

4. Mittel nach Anspruch 2, dadurch gekennzeichnet, daß das Nitrat Ammonium- oder Natriumnitrat ist.

5. Mittel nach Anspruch 3, dadurch gekennzeichnet, daß der UV-Absorber 2,2'-Dihydroxy-4-methoxybenzophenon und 2-Hydroxy-4-methoxybenzophenonsulfonsäure-(5) ist.

6. Verfahren zur Herstellung eines Mittels gemäß Anspruch 1, dadurch gekennzeichnet, daß ein Träger mit einer wäßrigen Lösung, die das Nitrat, gegebenenfalls den UV-Absorber, die Enzyme und das Chromogen enthält, nach einer Trocknung mit einer mit Wasser nicht mischbaren Lösung, die einen weiteren UV-Absorber und ein Hydrophobierungsmittel enthält, behandelt wird.

### Claims

1. An agent for detecting glucose in biological fluids, which essentially comprises an adsorbent matrix containing a chromogen, a glucose oxidase, a peroxidase and, optionally, a stabilizer, which contains a nitrate and, optionally, a UV absorber.

2. The agent as claimed in claim 1, wherein the nitrate is a nitrate of an element of the first group of the periodic table (1th main group 1a, CRC Handbook of Chemistry and Physics, 59th edition, CRC Press, 1979) or is ammonium nitrate.

3. The agent as claimed in claim 1, wherein the UV absorber is a derivative of benzoic acid, of benzophenone, of oxalic acid or is a benzotriazine.

4. The agent as claimed in claim 2, wherein the nitrate is ammonium or sodium nitrate.

5. The agent as claimed in claim 3, wherein the UV absorber is 2,2'-dihydroxy-4-methoxybenzophenone and 2-hydroxy-4-methoxybenzophenone-5-sulfonic acid.

6. A process for the preparation of an agent as claimed in claim 1, which comprises treating a support with an aqueous solution which contains the nitrate, optionally the UV absorber, the enzymes and the chromogen, and after drying, treating with a solution which is not miscible with water and which contains a further UV absorber and a water repelling agent.

### Revendications

1. Moyen pour la détection du glucose dans les liquides biologiques constitué essentiellement d'une matrice adsorbante contenant un chromogène, une glucose-oxydase, une peroxydase et éventuellement un stabilisant, caractérisé en ce qu'il contient un nitrate et éventuellement un absorbant des rayons UV.

2. Moyen selon la revendication 1, caractérisé en ce que le nitrate est un nitrate d'un élément du premier groupe de la classification périodique (premier groupe principal 1a. CRC Handbook of Chemistry and Physics 59" édition CRC Press 1978--1979) ou du nitrate d'ammonium.

3. Moyen selon la revendication 1, caractérisé en ce que l'absorbant d'UV est un dérivé de l'acide benzoïque, de la benzophénone, de l'acide oxalique ou une benzotriazine.

4. Moyen selon la revendication 2, caractérisé en ce que le nitrate est du nitrate d'ammonium ou du nitrate de sodium.

5. Moyen selon la revendication 3, caractérisé en ce que l'absorbant d'UV est de la 2,2'-dihydroxy-4-méthoxybenzophénone et de l'acide 2-hydroxy-4 méthoxy benzophénonesulfonique-(5).

6. Procédé de préparation d'un moyen selon la revendication 1, caractérisé en ce qu'un support imprégné d'une solution aqueuse qui contient le nitrate, éventuellement l'absorbant d'UV, les enzymes et le chromogène, est traitée après sé-

chage avec une solution non miscible à l'eau qui contient un autre absorbant d'UV et un agent d'hydrophobisation.